# NEW EUROPEAN PATENT SPECIFICATION

(11) **EP 3 122 385 B2**
(45) Date of publication and mention of the opposition decision: **15.06.2022**
(45) Mention of the grant of the patent: 14.11.2018
(21) Application number: 15715685.2
(22) Date of filing: 09.03.2015
(51) Int. Cl.: A61L 2/08, B29C 49/42, H01J 5/18

(54) **ELECTRON BEAM EMITTER**
ELEKTRONENSTRAHLEMITTER
ÉMETTEUR DE FAISCEAU D'ÉLECTRONS

(30) Priority: 24.03.2014 SE 1450334
(43) Date of publication of application: 01.02.2017
(73) Proprietor: Tetra Laval Holdings & Finance SA, 1009 Pully (CH)
(72) Inventor: HOSTETTLER, Urs, 3604 Thun (CH)
(74) Representative: Tetra Pak - Patent Attorneys SE
(86) International application number: PCT/EP2015/054814
(87) International publication number: WO 2015/144425

(56) References cited:
- EP-A1- 2 687 236
- EP-A1- 2 842 579
- EP-A1- 2 991 078
- WO-A1-2009/144114
- WO-A1-2014/095937
- GB-A- 277 347
- JP-A- 2003 307 598

## Description

This invention relates to an electron beam emitter, in particular for sterilization of packaging material, and to a sterilization device, in particular for packaging material.

Electron irradiation has been considered as a promising alternative for sterilizing purposes for which wet chemistry involving hydrogen peroxide has been the traditional technical platform. Known electron beam emitters use an electron generator for emitting charge carriers, such as electrons. The electron generator comprises a cathode housing and a filament. When an electrical current is set through the filament, an electrical resistance of the filament causes the filament to be heated which causes the filament further on to emit a cloud of electrons. The electrons leave the housing of the electron beam emitter via an electron exit window. During sterilization, the electron exit window heats up. Cooling methods or cooled electron exit windows are basically known from the prior art, for example through DE 20 2012 103 519 U1, WO 2009/144114 A1 and EP 1 982 921 B1.

It is an object of the current invention to provide an electron beam emitter, in particular for sterilization of packaging material, a sterilization device, in particular for packaging material and an insert for an electron beam emitter, in particular for sterilization a packaging material which provides an effective cooling of an electron exit window during sterilization and a flexible application of different cooling mediums.

This object is achieved by an electron beam emitter according to claim 1 and by a sterilization device according to claim 10. Additional advantages and features of preferred embodiments of the current invention are defined in the dependent claims.

The electron beam emitter, in particular for sterilization of packaging material, comprises a housing and an insert, wherein the housing comprises a first annular channel for guiding a medium, and wherein the first annular channel at least partially surrounds the insert, wherein the first annular channel is adapted to provide the medium, characterized in that the first annular channel is at least partly formed by the insert.

The insert is adapted to form part of an electron exit window. In other words, the insert is comprised in the electron exit window together with a foil element. According to one or more embodiments, the electron beam emitter is connected or connectable to a power supply unit. Generally, the power supply unit can also be connected to more than one electron beam emitters. The combination of the power supply unit and at least one electron beam emitter is named sterilization device. According to one or more embodiments the electron beam emitter comprises an electron generator for emitting charge carriers, such as electrons, along a path. The electron generator is generally enclosed in a hermetically sealed vacuum chamber. This vacuum chamber is one part of the aforementioned housing. The vacuum chamber is provided according to one or more embodiments with the electron exit window. Furthermore, the electron generator comprises a cathode housing and a filament. In use, an electron beam is generated by heating the filament. When an electrical current is set through the filament, the electrical resistance of the filament causes the filament to be heated to a temperature in the order of 2000 K. This heating causes the filament to emit a cloud of electrons. The electrons are accelerated towards the electron exit window by means of a high voltage potential between the cathode housing and the electron exit window. Subsequently, the electrons pass through the electron exit window and continue towards a target area, e. g. a part of the packaging material that has to be sterilized. The high voltage potential is created by connecting the cathode housing and the filament to the power supply unit and by connecting the vacuum chamber to ground. The voltage that is supplied by the power supply unit lies, according to one or more embodiments, within a range of about 80 to 150 kV. However, higher and lower values are also possible.

An electron beam emitter as described before can be used for sterilization of packaging material, packages for packaging food or drugs, food, biological or medical devices and so on. There are no limitations concerning the content of the packaging material. Thus, the content can be e. g. liquid or solid. There are also no limitations concerning the use of the sterilization device of the electron beam emitter itself, respectively. Thus, the electron beam emitter or the sterilization device, respectively, can be used for inside and/or outside sterilization of e. g. packaging material, such as packaging containers e. g. for food, liquids or drugs.

In use, in other words, during sterilization, the electron exit window and/or the insert, respectively, heat up. However, the first annular channel is advantageously adapted to provide the medium, in particular a cooling medium, such as for example water or a specific coolant. The medium that is used to cool the insert can be liquid or gaseous. In this context, it is an advantage that the first annular channel that at least partially surrounds the insert is a closed annular channel. Thus, the cooling medium is guided around the insert wherein the cooling medium absorbs heat from the insert and wherein the cooling medium transfers the heat away from the insert. There is no possibility that cooling medium could leave or drop out of the first annular channel. As a consequence, there is no risk that material that has to be sterilized is polluted or damaged by the cooling medium. The design of the insert optimizes the heat transfer to the cooling medium as the first annular channel is at least partly formed by the insert itself. Thus, the cooling medium and the element that has to be cooled, in this case the insert, are in direct contact. This means, for example, that there are no walls in between that could e. g. interrupt the heat transfer.

According to one or more embodiments, the insert is for example made of copper which provides very high heat conductivity. It goes without saying that also other materials, in particular metals or fibre reinforced materials, which provide high heat conductivity can be also used. A mass flow of the (liquid cooling) medium lies according to one ore more embodiments within a range of about 3-5 l/min. A temperature of the insert and in particular of the support structure lies expediently within a range of about 150 to 230 °C.

According to one or more embodiments, the housing comprises a first body and a second body, wherein the first body comprises the cathode housing and the filament and wherein the insert is arranged at the second body. Cross sections of the two bodies are expediently round, in particular circular, wherein a diameter of the first body is bigger than a diameter of the second body. According to one or more embodiments the first body comprises the cathode housing and the filament. The second body comprises the electron exit window. Expediently, the second body has a longitudinal shape, such as a cylindrical shape, which allows an insertion e. g. into a packaging container, such as a packaging container made of carton-based packaging laminate or a solely polymeric material such as for instance PET. The diameter of the first body is preferably bigger which minimizes the risk of creating electrical arcs inside the housing. The above mentioned vacuum chamber is formed by the second body and at least partly by the first body. According to one or more embodiments the first body is adapted to be connected to the power supply unit e. g. via a high voltage output connector of the at least one power supply unit. In general, a plurality of sterilization devices is arranged at a movable or rotatable carousel or carrier plate.

According to one or more embodiments, the insert comprises a support structure and a wall structure, wherein the wall structure forms at least partly the first annular channel. Basically, the housing comprises walls or is, in other words, formed by walls. According to one or more embodiments, the insert is arranged at and/or in the housing. Expediently, the wall structure of the insert is adapted to continue a design of the wall(s) of the housing. For that case, the insert and in particular the wall structure of the insert comprises an end portion that is arranged or arrangeable, respectively, at the housing. As a consequence, the form and design of the housing or its walls can be continued by the insert. This means for example that an inner surface of the housing is formed by the walls of the housing and by the insert or the wall structure of the insert, respectively. On the other side, the wall structure is adapted to form the first annular channel together with the housing. Generally speaking, the insert or the wall structure of the insert, respectively, is adapted to continue a cross section of that part of the wall of the housing, that the end portion of the wall structure is arranged at.

Generally, the housing extends along a longitudinal axis. The longitudinal axis correlates basically to a direction of the aforementioned electron path. The wall structure is according to one or more embodiments formed as a ring, wherein the ring has an extension along the longitudinal axis. As a consequence, the first annular channel has also an extension along the longitudinal axis. The first annular channel can be even higher as for example the wall structure. In this case, at least parts of the first annular channel are only formed by the housing or by the walls of the housing, respectively. According to one or more embodiments, the support structure is formed as a disc-shaped grid. The support structure is basically orientated with its centre axis aligned with the longitudinal axis of the electron beam emitter, i.e. a plane corresponding to the disc surface is oriented perpendicular to the wall structure. The wall structure is a round or circular element, wherein an inner space of the ring is covered or filled with the support structure. Expediently, the support structure forms an end portion of the housing when the insert is arranged at and/or in the housing. The main purpose of the support structure is to form a support for the foil element.

According to one or more embodiments, the electron beam emitter comprises the foil element, wherein the foil element is arranged at the insert. In other words, the foil element is adapted to form a boundary of the housing or a boundary of the internal vacuum space of the housing, respectively. The foil element can be for example made of aluminum, copper, titanium or zirconium or by a combination of at least some of these materials. A thickness of the foil element (measured along the longitudinal axis) lies within a range of about 8 to 12 µm. Thus, due to these dimensions it is very difficult to fix the foil element at another element. Therefore, the insert comprises the support structure that has, as already mentioned, advantageously the form of a grid or a web. The support structure also comprises a plurality of openings or holes, such as round, oval, circular or polygonal openings. The foil element is adapted to hold or keep the vacuum inside the housing, and at the same time be transparent to electrons. The same is valid for the design of the support structure. Thus, the support structure has the grid-like or web-like design which provides on the one hand a big area of support for arranging the foil element and on the other hand, due to the grid-like or web-like design, a very lean form that allows the electrons to pass the support structure without hitting it. In addition, the permeable or perforated design or form of the support structure allows a pulling or drawing of the foil element via the vacuum that is inside the housing. Thus, a very strong and durable arrangement can be achieved. According to one or more embodiments the foil element is cooled down to a temperature that lies within a range of about 150 to 200 °C.

According to one aspect of the invention, the partition wall basically surrounds the wall structure. The partition wall extends a distance in a radial direction and extends along the perimeter of the wall structure. Thus, the partition wall extends basically perpendicular to the wall structure. If the insert is arranged at and/or in the housing, the partition wall expediently extends from the wall structure to the housing, in particular to an outer wall of the housing. A plurality of annular channels can be formed by providing an appropriate number of partition walls oriented next to each other along the longitudinal axis of the housing, i.e. the partition walls are oriented as rings one after the other along the axis forming channels there between. Expediently, the partition walls comprise one or more openings so that a connection can be realized between the different annular channels. According to one aspect of the invention, the different annular channels can have different sizes and/or volumes. As a consequence, the cooling performance can be adjusted via the volume of the annular channels.

As already mentioned, the housing or the second body, respectively, comprises the vacuum. Advantageously, due to the connection of the internal space to the second annular channel, the vacuum can also be realized within the second annular channel. Thus, a possible arrangement area for the foil element that works with the vacuum-effect as described before can be increased. It goes without saying that in this case the partition wall should not comprise an opening to the first annular channel.

According to one or more embodiments, the foil element is arranged at a retainer, wherein the retainer is arrangeable or arranged at the housing and/or at the insert. According to one or more embodiments, the retainer is formed like a round, in particular a circular, ring or frame, wherein the foil element is attached at the ring. This allows a very secure handling of the sensitive foil element and in addition a very easy arrangement at the housing and/or the insert.

The housing comprises at least one inlet channel and at least one outlet channel. The inlet channel is adapted to supply at least the first annular channel with the medium, in particular the cooling medium such as water or the coolant medium, whereas the outlet channel is adapted to drain the medium from the first annular channel. Expediently, the inlet and the outlet channel or channels have the same design. As a consequence, an inlet channel can be also used as outlet channel and vice versa. Thus a flow direction of the medium can be easily changed. According to one aspect of the invention, the channels are oriented basically along the longitudinal axis. They can extend parallel to the longitudinal axis. However, also a curved form, e. g. a helical form, is possible. Expediently, the design of the inlet channel as well as the outlet channel are continued by the first annular channel or fade to the first annular channel. Generally speaking, the inlet channel and the outlet channel are connected to the first annular channel so that the cooling medium can be provided. The inlet and outlet channels debouche at mutual opposite positions in the first annular channel such that the medium, upon entering the first annular channel from the inlet channel, will be divided into two flows one taking a clockwise direction and the other a counterclockwise direction towards the outlet channel. Expediently, the housing comprises the connection ports which enable a very simple and flexible supply of the cooling medium. According to one or more embodiments, the inlet and outlet channels are located inside or within the walls of the housing. Thus, the channels can be realized by appropriate bores or holes that are drilled e. g. along the longitudinal axis of the housing. In this context, it has to be mentioned that the connection ports are expediently positioned or arranged at the second body or at least near to the second body. According to an aspect of the invention, the electron beam emitter and in particular its end portion which comprises the electron exit window is inserted into a packaging container or vice versa. Thus, the bottom part of the housing should be free of any connection ports etc. As a consequence, the medium supply has to be realized from above e. g. via the inlet and outlet channels that are directed along the longitudinal axis.

According to one or more embodiments, the housing comprises at least one cover plate, wherein the at least one cover plate is adapted to form an outer surface of the housing. In addition, the cover plate is adapted to form at least partly the inlet and/or the outlet channel. Expediently, a profile or a contour can be milled along the longitudinal axis on an outer surface of the housing. The profile or contour can be covered by the appropriate designed cover plate so that the inlet channels and outlet channels are formed. As the cover plates are adapted to form the outer surface of the housing, the outer diameter of the housing is not changed. This can be a much cheaper solution as e. g. drilling the inlet or outlet channels.

According to one or more embodiments, the housing comprises a tubular element, wherein the tubular element and the insert form the first annular channel. The tubular element is arranged coaxially with the insert a distance outside of the perimeter of the insert. Similar to the cover plates, the tubular ring expediently forms an outer surface of the housing. In particular, a seamless outer surface is formed by the cover plates and the tubular element in combination with the housing itself. The use of the tubular element allows a very cost effective construction of the electron beam emitter as it can be easily pushed over the insert to form the at least one annular channel.

The sterilization device, in particular for packaging material, comprises a power supply unit and at least one electron beam emitter, wherein the electron beam emitter comprises a housing and an insert, wherein the housing comprises a first annular channel and wherein the first annular channel surrounds the insert, wherein the first annular channel is adapted to provide a medium, characterized in that the first annular channel is at least partly formed by the insert.

According to another aspect of the invention, an insert for an electron beam emitter, in particular for sterilization of packaging material, comprises a support structure and a wall structure, wherein the support structure is adapted for an arrangement of a foil element, characterized in that the wall structure and a partition wall form at least partly a first annular channel.

The electron beam emitter according to the invention can include the features and advantages of the sterilization device according to the invention and vice versa.

Additional aspects and features of the current invention are shown in the following description of preferred embodiments of the current invention with reference to the attached drawings. Single features or characteristics of respective embodiments are explicitly allowed to be combined within the scope of the current invention.
- Figure 1:: shows a principle scheme of an embodiment of an electron beam emitter;
- Figure 2:: shows a cross section of a second body of an electron beam emitter;
- Figure 3:: shows an embodiment of an insert from an inside of a housing;
- Figure 4:: shows an embodiment of an insert arranged at a housing;
- Figure 5a:: shows an intersection plane for the sectioning of Figure 5b;
- Figure 5b:: shows the sectioning as indicated in Figure 5a;
- Figure 6:: shows a further embodiment of an electron beam emitter;

- Figure 7:: shows the arrangement of an insert at a housing;
- Figure 8:: shows a further embodiment of an insert and its arrangement at a housing.

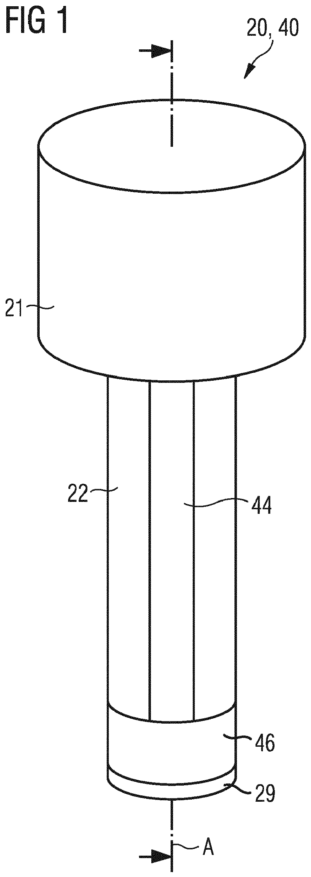

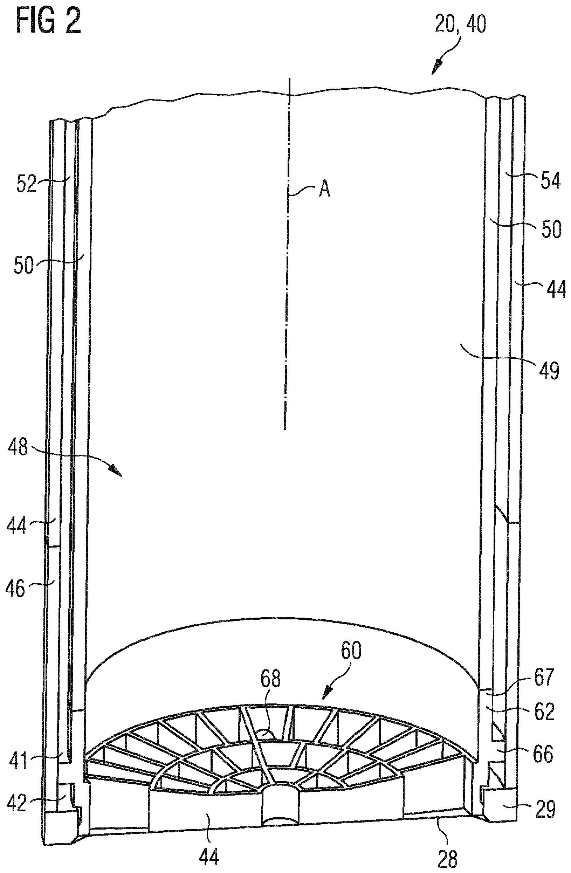

Referring now to **Figure 1**, an electron beam emitter 20 is shown wherein the electron beam emitter 20 comprises a housing 40. The housing 40 comprises a first body 21 and a second body 22. Both bodies 21, 22 extend along a longitudinal centre axis A. Cross sections of the first body 21 and the second body 22 are basically round, in particular circular, wherein a diameter of the first body is bigger than a diameter of the second body 22. According to one or more embodiments, the first body 21 comprises a cathode housing and a filament (not shown in Figure 1). Amongst others, the bigger diameter of the first body 21 which comprises the above named components decreases the risk of electric arcs. According to one or more embodiments, the first body 21 is connected or connectable, respectively, to a power supply unit (not shown in Figure 1). The second body 22 and at least a part of the first body 22 comprise a vacuum, i.e. form a vacuum chamber. The second body 22 comprises cover plates 44, a tubular element 46 and a retainer 29 that forms an end of the housing 40. A sectional view of the second body 22 that is indicated by the small arrows is explained in Figure 2.

**Figure 2** shows a lower part of the second body 22 of the housing 40. An insert 60 is arranged at an end portion of the housing 40. In particular, an end portion 67 of the insert 60 is arranged at a wall 50 of the housing 40. In other words, the shape of the cross section of the wall 50 is continued by a wall structure 62 of the insert 60. Hence, the wall structure 62 is annular, i.e. formed as a sleeve or a shell with an extension along longitudinal axis A. The insert 60 comprises also a disc-shaped support structure 64 that has a radial extension being basically perpendicular to the longitudinal axis A of the housing 40. The wall structure 62 of the insert 60 forms at least partly a first annular channel 41. The first annular channel 41 is also formed by a tubular element 46 that is arranged at the wall 50. Cover plates 44 and the wall 50 of the housing 40 form inlet and outlet channels 52, 54 that extend basically along the longitudinal axis A. The inlet and outlet channels 52, 54 are connected to the first annular channel 41 at mutual opposite locations. The first annular channel 41 is limited along the longitudinal axis A by a partition wall 66 that basically surrounds the perimeter of the wall structure 62. The partition wall 66 forms at least partly a second annular channel 42. The second annular channel 42 is connected to an internal space 48 of the housing 40 via an opening 68 that is arranged at and/or in the insert 60. The insert 60 or its wall structure 62, respectively, forms a seamless inner surface 49. Due to the connection of the internal space 48 to the second annular channel 42 via the opening 68 a vacuum that is inside the housing 40 is also in the second annular channel 42. Therefore, a foil element 28 and a retainer 29 can be optimally arranged at the support structure 62 and at the housing 40 or the tubular element 46, respectively. The foil is preferably bonded to an outer surface of the retainer, e.g. by diffusion bonding. The outer surface of the retainer 29 is arranged at a distance, along the longitudinal axis A, from the outer surface of the support structure 62. Thereby, the bonding line of the foil is formed on a plateau, i.e. elevated, compared to the outer surface of the support structure 62.

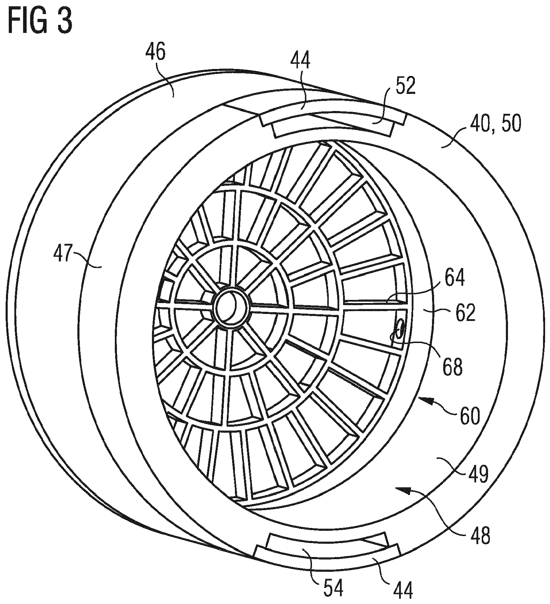

**Figure 3** shows an end portion of a housing 40. In particular, an insert 60 is shown from an inside of the housing 40. The housing 40 is formed by a wall 50, wherein inlet and outlet channels 52, 54 are formed by the wall 50 and appropriate cover plates 44. The cover plates 44 and the wall 50 form an outer surface 47 of the housing 40. In the same way, the outer surface 47 is continued by a tubular element 46 that surrounds the insert 60. The insert 60 comprises a support structure 64 and a wall structure 62, wherein the wall structure 62 and the wall 50 form an inner surface 49 of the housing 40. An internal space 48 of the housing 40 is connected to a second annular channel (not visible) via at least one opening 68.

The tubular element 46 may be an integrated, i.e. in one piece formed, portion of the rest of the housing 40.

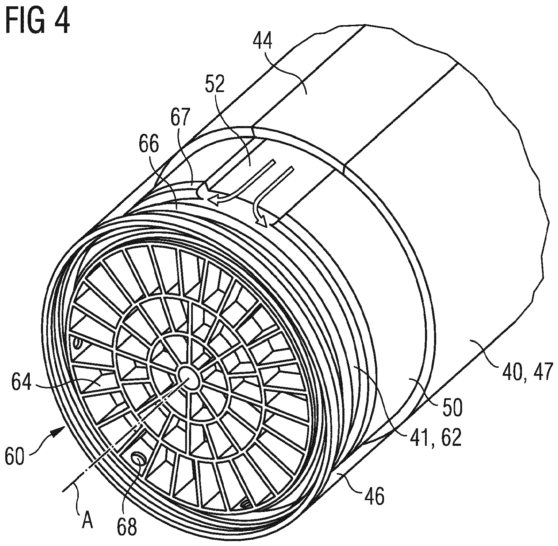

**Figure 4** shows an embodiment of an insert 60 that is arranged at the housing 40. A tubular element 46 is arranged at the housing 40. However, the tubular element 46 is shown transparent so that a shape of an inlet channel 52 can be seen. The outlet or inlet channel 52, 54 is connected to a first annular channel 41 that is formed by the insert 60 and in particular by a wall structure 62 of the insert 60. The outlet channel is not visible in Figure 4 since it is being located opposite the inlet channel 52. The flow of the medium entering the first annular channel 41, from the inlet channel 52, will be divided into two flows, one directed clockwise towards the outlet channel and the other directed counterclockwise towards the outlet channel. Arrows illustrate this. An end portion 67 continues the form and design of a wall 50 of the housing 40. The inlet or outlet channel 52, 54 is formed by the wall 50 of the housing 40 and a cover plate 44. The cover plate 44 forms together with the wall 50 of the housing 40 an outer surface 47. The insert 60 comprises a partition wall 66 that is adapted to form at least partly a second annular channel 42 (see Figure 2), wherein a connection from an internal space of the housing 40 to the second annular channel 42 can be realized by a plurality of openings 68. The insert 60 comprises a grid- or web-like support structure 64.

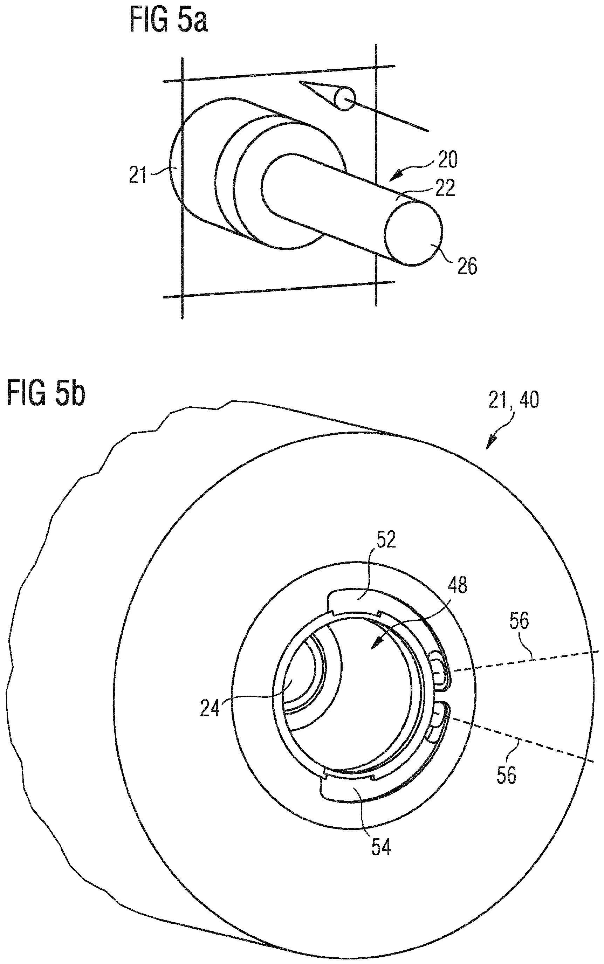

**Figure 5a** just shows an intersection plane of an embodiment of an electron beam emitter 20 comprising a first body 21 and a second body 22, wherein the second body 22 comprises an electron exit window 26. The small arrow indicates a viewing direction for the sectioning shown in Figure 5b.

**Figure 5b** shows a filament 24 that is arranged inside the first body 21 of a housing 40. Two semicircular pockets are shown that merge to the already known shape of the inlet and outlet channels 52, 54, as for example shown in Figure 4. This allows a basically perpendicular deflection of a (cooling) medium flow from connection ports 56 to the inlet and outlet channels 52, 54 respectively. The connection ports 56 are indicated by the dotted lines.

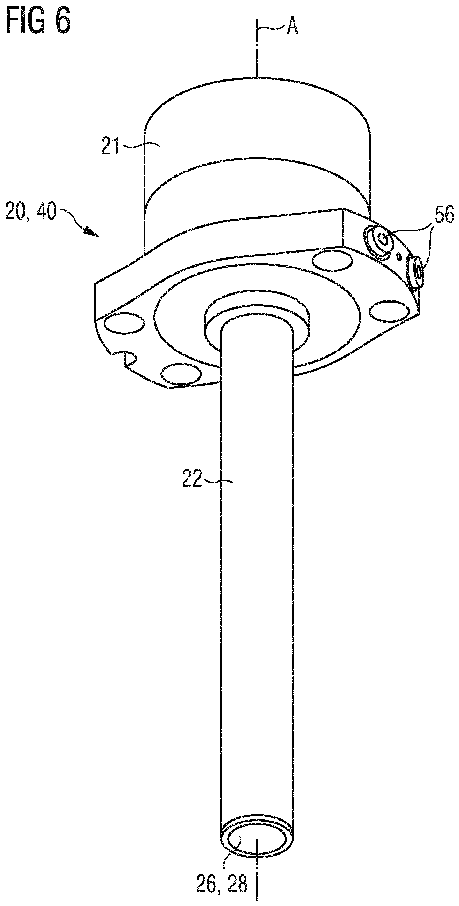

**Figure 6** shows a further embodiment of an electron beam emitter 20 comprising a housing 40, wherein the housing 40 comprises a first body 21 and a second body 22. The housing 40 extends along a longitudinal axis A, wherein the second body 22 comprises an electron exit window 26 with a foil element 28 at its end portion. Between the first body 21 and the second body 22 there is arranged a kind of flange, wherein the flange comprises two connection ports 56 that are adapted to supply a cooling medium. In addition, the flange comprises four openings or holes that can be used to arrange the electron beam emitter for example at a movable or rotatable carousel or carrier plate.

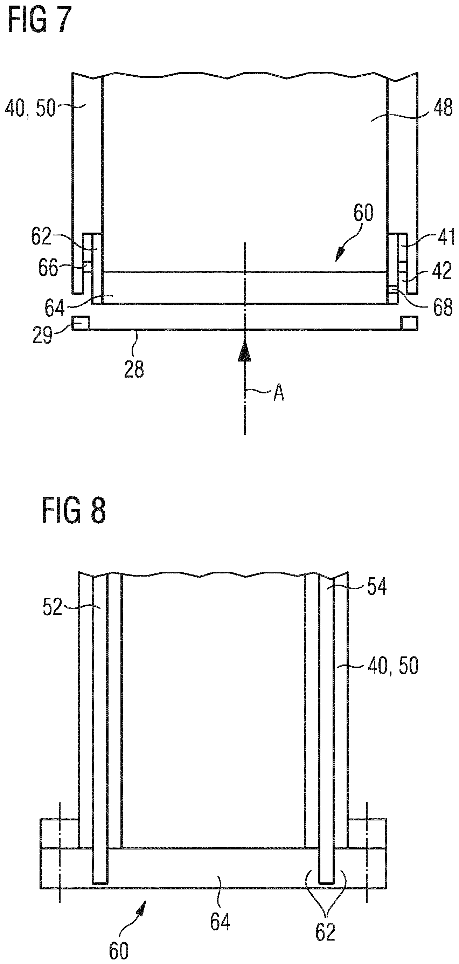

**Figure 7** shows a principle scheme of an arrangement of an insert 60 at a housing 40. The housing 40 comprises a wall 50 (without cover plates). The insert 60 comprises a wall structure 62 and a support structure 64. A first annular channel 42 is formed by the insert 60 and in particular by the wall structure 62 and by a partition wall 66. The partition wall 66 forms furthermore a second annular channel 42 that is connected via an opening 68 to an internal space 48 of the housing 40. A foil element 28 and a retainer 29 can be easily arranged at the insert 60 and the housing 40 e.g. moving it along the direction of the small arrow that extends along the longitudinal axis A.

**Figure 8** shows a further embodiment of an insert 60 that comprises a support structure 64 and a wall structure 62. However, the arrangement at a housing 40 that comprises inlet and outlet channels 52, 54 is different. In particular, the housing 40 forms a kind of flange, wherein the insert 60 can be arranged at the flange via appropriate fixing material, e. g, indicated by the dashed lines. A foil element and its arrangement are not shown in Figure 8.

The electron beam emitter may be connected to a conditioning system as described in the Swedish Patent Application No. SE 1450217-3 filed by the applicant. In such system the cooling medium is first used to cool a power supply unit connected to the electron beam emitter. Hence, the flow direction of the medium is directed from the power supply unit to the electron beam emitter. Generally, the temperature level of the power supply unit is lower than a temperature level of the electron beam emitter. This means that the medium flow that has already been heated up during cooling of the power supply unit can still be used for cooling the electron beam emitter and in particular its electron exit window. Further, it is an advantage if the electron exit window is cooled with a medium flow that is warmer than an ambient temperature of the electron exit window. In that way condensation on the electron exit window may be avoided.

The electron beam emitter 20 according to the invention can be arranged in an irradiation chamber in a filling machine. The filling machine comprises at least one filling station for filling content into the packaging container and at least one station for sealing the opening after filling. The electron beam emitter can for example be applied in the application described in the international application No. PCT/EP2013/076870 filed by the applicant. A plurality of emitters can be provided on a carousel or the like which is adapted to rotate. The emitters may be arranged in holes in the carousel. The packaging containers, which are transported for example via a conveyor, reach the carousel and are engaged with one of the (rotating) emitters for interior surface sterilization. During at least a part of one rotation of the carousel the interior sterilization takes place. During interior sterilization a relative movement is created between the packaging container and the electron beam emitter, in particular the packaging containers are lifted to surround the electron beam emitters such that the electron cloud emitted through the electron exit window is inserted into the packaging container and can reach the interior surface thereof. After sterilization the packaging container is removed from the emitter or from the carousel, respectively. The packaging container is then subsequently transported through an electron cloud provided in a gap between two emitters for outside surface sterilization.

### Reference numerals

- 20: electron beam emitter
- 21: first body
- 22: second body
- 24: filament
- 26: electron exit window
- 28: foil element
- 29: retainer
- 40: housing
- 41: first annular channel
- 42: second annular channel
- 44: cover plate
- 46: tubular element
- 47: outer surface
- 48: internal space
- 49: inner surface
- 50: wall
- 52: inlet channel
- 54: outlet channel
- 56: connection port
- 60: insert
- 62: wall structure
- 64: support structure
- 66: partition wall
- 67: end portion
- 68: opening
- A: longitudinal axis

## Claims

1. Electron beam emitter (20), in particular for sterilization of packaging material,
comprising a housing (40), an electron exit window (26) and an insert (60), wherein the housing (40) comprises a first annular channel (41) for guiding a medium, and
wherein the first annular channel (41) at least partially surrounds the insert (60) and is adapted to provide the medium,
**characterized in that**
the first annular channel (41) is at least partly formed by the insert (60), wherein the insert (60) is adapted to form part of the electron exit window (26),
wherein the housing (40) comprises at least one inlet channel (52) and at least one outlet channel (54), and wherein the outlet and inlet channel (52, 54) are connected to and in fluid connection with the first annular channel (41), and which are adapted to supply the medium to the first annular channel (41),
wherein the housing (40) comprises at least one connection port (56) for the inlet channel (52) and at least one connection port (56) for the outlet channel (54), said connection ports being connected to a medium supply, and wherein the insert (60) comprises a partition wall (66),
wherein the partition wall (66) forms at least partly a second annular channel (42), and
wherein the insert (60) comprises at least one opening (68), and
wherein the at least one opening (68) is adapted to provide a connection between an internal space (48) of the housing (40) and the second annular channel (42).

2. Electron beam emitter (20) according to claim 1,
wherein the housing (40) comprises a first body (21) and a second body (22),
wherein the first body (21) comprises a cathode housing and a filament (24), and
wherein the insert (60) is arranged at the second body (22).

3. Electron beam emitter (20) according to any of the preceding claims,
wherein the insert (60) comprises a support structure (64) and a wall structure (62),
wherein the wall structure (62) forms at least partly the first annular channel (41).

4. Electron beam emitter (20) according to any of the preceding claims, wherein the insert (60) comprises a support structure (64), wherein the electron beam emitter comprises a foil element (28), and wherein the foil element (28) is arranged to be supported by the support structure.

5. Electron beam emitter (20) according to any of the preceding claims,
wherein the foil element (28) is arranged at a retainer (29), and
wherein the retainer (29) is arranged at the housing (40).

6. Electron beam emitter (20) according to any of the preceding claims,
wherein the housing (40) comprises at least one cover plate (44),
wherein the at least one cover plate (44) is adapted to form an outer surface (47) of the housing (40) and to at least partly form the inlet and outlet channel.

7. Electron beam emitter (20) according to any of the preceding claims,
wherein the housing (40) comprises a tubular element (46),
wherein the tubular element (46) and the insert (60) form the first annular channel (41).

8. Electron beam emitter (20) according to any one of the preceding claims,
wherein the inlet and outlet channels (52, 54) debouche at mutual opposite positions in the first annular channel (41) such that the medium, upon entering the first annular channel from the inlet channel (52), will be divided into two flows one taking a clockwise direction and the other a counterclockwise direction towards the outlet channel (54).

9. Electron beam emitter (20) according to any of the preceding claims, wherein the medium is water or other liquid coolant.

10. Sterilization device, in particular for packaging material,
comprising a power supply unit and at least one electron beam emitter (20) according to claim 1,
wherein the electron beam emitter (20) comprises a housing (40) and an insert (60),
wherein the housing (40) comprises a first annular channel (41), and wherein the first annular channel (41) surrounds the insert (60),
wherein the first annular channel (41) is adapted to provide a medium, **characterized in that**
the first annular channel (41) is at least partly formed by the insert (60), wherein the insert (60) is adapted to form part of an electron exit window (26) and wherein the housing (40) comprises at least one inlet channel (52) and at least one outlet channel (54), wherein the outlet and inlet channel (52, 54) is connected to the first annular channel (41).

## Patentansprüche

1. Elektronenstrahlemitter (20), insbesondere zum Sterilisieren von Verpackungsmaterial,
umfassend ein Gehäuse (40), ein Elektronenaustrittsfenster (26) und einen Einsatz (60),
wobei das Gehäuse (40) einen ersten ringförmigen Kanal (41) zum Führen eines Mediums umfasst, und wobei der erste ringförmige Kanal (41) den Einsatz (60) mindestens teilweise umgibt und vorgesehen ist, um das Medium bereitzustellen,
**dadurch gekennzeichnet, dass**
der erste ringförmige Kanal (41) mindestens teilweise durch den Einsatz (60) gebildet ist, wobei der Einsatz (60) vorgesehen ist, um Teil des Elektronenaustrittsfensters (26) zu bilden, wobei das Gehäuse (40) mindestens einen Einlasskanal (52) und mindestens einen Auslasskanal (54) umfasst, und wobei der Auslass- und Einlasskanal (52, 54) mit dem ersten ringförmigen Kanal (41) verbunden und in Fluidverbindung mit dem ersten ringförmigen Kanal (41) sind, und die vorgesehen sind, um dem ersten ringförmigen Kanal (41) das Medium zuzuführen, und
wobei das Gehäuse (40) mindestens einen Verbindungsanschluss (56) für den Einlasskanal (52) und mindestens einen Verbindungsanschluss (56) für den Auslasskanal (54) umfasst, wobei die Verbindungsanschlüsse mit einer Zufuhr für das Medium verbunden sind und
wobei der Einsatz (60) eine Trennwand (66) umfasst,
wobei die Trennwand (66) mindestens teilweise einen zweiten ringförmigen Kanal (42) bildet und wobei der Einsatz (60) mindestens eine Öffnung (68) umfasst, und wobei die mindestens eine Öffnung (68) vorgesehen ist, um eine Verbindung zwischen einem Innenraum (48) des Gehäuses (40) und dem zweiten ringförmigen Kanal (42) bereitzustellen.

2. Elektronenstrahlemitter (20) nach Anspruch 1,
wobei das Gehäuse (40) einen ersten Körper (21) und einen zweiten Körper (22) umfasst,
wobei der erste Körper (21) ein Kathodengehäuse und ein Filament (24) umfasst, und
wobei der Einsatz (60) an dem zweiten Körper (22) angeordnet ist.

3. Elektronenstrahlemitter (20) nach einem der vorhergehenden Ansprüche,
wobei der Einsatz (60) eine Trägerstruktur (64) und eine Wandstruktur (62) umfasst,
wobei die Wandstruktur (62) mindestens teilweise den ersten ringförmigen Kanal (41) bildet.

4. Elektronenstrahlemitter (20) nach einem der vorhergehenden Ansprüche, wobei der Einsatz (60) eine Trägerstruktur (64) umfasst, wobei der Elektronenstrahlemitter ein Folienelement (28) umfasst, und wobei das Folienelement (28) so angeordnet ist, dass es von der Trägerstruktur getragen wird.

5. Elektronenstrahlemitter (20) nach einem der vorhergehenden Ansprüche, wobei das Folienelement (28) in einem Halter (29) angeordnet ist, und wobei der Halter (29) an dem Gehäuse (40) angeordnet ist.

6. Elektronenstrahlemitter (20) nach einem der vorhergehenden Ansprüche, wobei das Gehäuse (40) mindestens eine Abdeckplatte (44) umfasst,
wobei die mindestens eine Abdeckplatte (44) vorgesehen ist, um eine Außenfläche (47) des Gehäuses (40) zu bilden und um mindestens teilweise den Einlass- und Auslasskanal zu bilden.

7. Elektronenstrahlemitter (20) nach einem der vorhergehenden Ansprüche,
wobei das Gehäuse (40) ein rohrförmiges Element (46) umfasst,
wobei das rohrförmige Element (46) und der Einsatz (60) den ersten ringförmigen Kanal (41) bilden.

8. Elektronenstrahlemitter (20) nach einem der vorhergehenden Ansprüche,
wobei die Einlass- und Auslasskanäle (52, 54) sich an einander gegenüber liegenden Positionen in den ersten ringförmigen Kanal (41) ergießen, so dass das Medium nach Eintreten in den ersten ringförmigen Kanal aus dem Einlasskanal (52) in zwei Flüsse geteilt wird, von denen einer die Richtung im Uhrzeigersinn und der andere eine Richtung gegen den Uhrzeigersinn in Richtung des Auslasskanals (54) nimmt.

9. Elektronenstrahlemitter (20) nach einem der vorhergehenden Ansprüche, wobei das Medium Wasser oder anderes flüssiges Kühlmittel ist.

10. Sterilisiervorrichtung, insbesondere für Verpackungsmaterial,
umfassend eine Stromversorgungseinheit und mindestens einen Elektronenstrahlemitter (20) nach Anspruch 1,
wobei der Elektronenstrahlemitter (20) ein Gehäuse (40) und einen Einsatz (60) umfasst,
wobei das Gehäuse (40) einen ersten ringförmigen Kanal (41) umfasst, und
wobei der erste ringförmige Kanal (41) den Einsatz (60) umgibt,
wobei der erste ringförmige Kanal (41) vorgesehen ist, um ein Medium bereitzustellen, **dadurch gekennzeichnet, dass**
der erste ringförmige Kanal (41) mindestens teilweise durch den Einsatz (60) gebildet ist, wobei der Einsatz (60) vorgesehen ist, um Teil des Elektronenaustrittsfensters (26) zu bilden, und wobei das Gehäuse (40) mindestens einen Einlasskanal (52) und mindestens einen Auslasskanal (54) umfasst, und wobei der Auslassund Einlasskanal (52, 54) mit dem ersten ringförmigen Kanal (41) verbunden sind.

## Revendications

1. Émetteur de faisceau d'électrons (20), en particulier pour la stérilisation de matériau d'emballage,
comprenant un boîtier (40), une fenêtre de sortie d'électrons (26) et une pièce rapportée (60),
dans lequel le boîtier (40) comprend un premier canal annulaire (41) destiné à guider un milieu, et
dans lequel le premier canal annulaire (41) entoure au moins partiellement la pièce rapportée (60) et est adapté pour fournir le milieu,
**caractérisé en ce que**
le premier canal annulaire (41) est au moins partiellement formé par la pièce rapportée (60),
dans lequel la pièce rapportée (60) est adaptée pour former une partie de la fenêtre de sortie d'électrons (26),
dans lequel le boîtier (40) comprend au moins un canal d'entrée (52) et au moins un canal de sortie (54), et dans lequel les canaux de sortie et d'entrée (52, 54) sont raccordés au et en raccordement fluidique avec le premier canal annulaire (41), et qui sont adaptés pour fournir le milieu au premier canal annulaire (41),
dans lequel le boîtier (40) comprend au moins un orifice de raccordement (56) pour le canal d'entrée (52) et au moins un orifice de raccordement (56) pour le canal de sortie (54), lesdits orifices de raccordement étant raccordés à une source de milieu, et
dans lequel la pièce rapportée (60) comprend une paroi de séparation (66),
dans lequel la paroi de séparation (66) forme au moins partiellement un deuxième canal annulaire (42),
et
dans lequel la pièce rapportée (60) comprend au moins une ouverture (68), et
dans lequel l'au moins une ouverture (68) est adaptée pour fournir un raccordement entre un espace interne (48) du boîtier (40) et le deuxième canal annulaire (42).

2. Émetteur de faisceau d'électrons (20) selon la revendication 1,
dans lequel le boîtier (40) comprend un premier corps (21) et un deuxième corps (22),
dans lequel le premier corps (21) comprend un boîtier de cathode et un filament (24), et
dans lequel la pièce rapportée (60) est disposée au niveau du deuxième corps (22).

3. Émetteur de faisceau d'électrons (20) selon l'une quelconque des revendications précédentes,
dans lequel la pièce rapportée (60) comprend une structure de support (64) et une structure de paroi (62),
la structure de paroi (62) formant au moins partiellement le premier canal annulaire (41).

4. Émetteur de faisceau d'électrons (20) selon l'une quelconque des revendications précédentes, dans lequel la pièce rapportée (60) comprend une structure de support (64), l'émetteur de faisceau d'électrons comprenant un élément en feuille (28), et l'élément en feuille (28) étant disposé pour être supporté par la structure de support.

5. Émetteur de faisceau d'électrons (20) selon l'une quelconque des revendications précédentes,
dans lequel l'élément en feuille (28) est disposé au niveau d'un dispositif de retenue (29), et
dans lequel le dispositif de retenue (29) est disposé au niveau du boîtier (40).

6. Émetteur de faisceau d'électrons (20) selon l'une quelconque des revendications précédentes,
dans lequel le boîtier (40) comprend au moins une plaque de recouvrement (44),
l'au moins une plaque de recouvrement (44) étant adaptée pour former une surface externe (47) du boîtier (40) et pour former au moins partiellement le canal d'entrée et de sortie.

7. Émetteur de faisceau d'électrons (20) selon l'une quelconque des revendications précédentes,
dans lequel le boîtier (40) comprend un élément tubulaire (46),
l'élément tubulaire (46) et la pièce rapportée (60) formant le premier canal annulaire (41).

8. Émetteur de faisceau d'électrons (20) selon l'une quelconque des revendications précédentes, dans lequel les canaux d'entrée et de sortie (52, 54) débouchent à des positions mutuellement opposées dans le premier canal annulaire (41) de telle sorte que le milieu, à son entrée dans le premier canal annulaire depuis le canal d'entrée (52), sera divisé en deux écoulements, l'un prenant une direction horaire et l'autre une direction antihoraire vers le canal de sortie (54).

9. Émetteur de faisceau d'électrons (20) selon l'une quelconque des revendications précédentes, dans lequel le milieu est l'eau ou un autre réfrigérant liquide.

10. Dispositif de stérilisation, en particulier pour matériau d'emballage,
comprenant une unité d'alimentation électrique et au moins un émetteur de faisceau d'électrons (20) selon la revendication 1,
dans lequel l'émetteur de faisceau d'électrons (20) comprend un boîtier (40) et une pièce rapportée (60),
dans lequel le boîtier (40) comprend un premier canal annulaire (41), et
dans lequel le premier canal annulaire (41) entoure la pièce rapportée (60),
le premier canal annulaire (41) étant adapté pour fournir un milieu,
**caractérisé en ce que**
le premier canal annulaire (41) est au moins partiellement formé par la pièce rapportée (60),
dans lequel la pièce rapportée (60) est adaptée pour former une partie d'une fenêtre de sortie d'électrons (26), et dans lequel le boîtier (40) comprend au moins un canal d'entrée (52) et au moins un canal de sortie (54), le canal de sortie et d'entrée (52, 54) étant raccordé au premier canal annulaire (41) .
